# EUROPEAN PATENT APPLICATION

(11) **EP 3 556 841 A1**
(43) Date of publication of application: **23.10.2019**
(21) Application number: 18168281.6
(22) Date of filing: 19.04.2018
(51) Int. Cl.: C12M 1/00, C12M 3/00

(54) **DISPOSABLE CARTRIDGE COOPERATING WITH A PLATFORM IN A FLEXIBLE SYSTEM FOR HANDLING AND/OR MANIPULATING FLUIDS**

(71) Applicant: Aglaris Ltd, Stevenage, Hertfordshire SG1 2FX (GB)
(72) Inventor: HORNA TOMÁS, David, Stevenage, Hertfordshire SG1 2FX (GB); COSTA FERRANDO, Miquel, Stevenage, Hertfordshire SG1 2FX (GB)
(74) Representative: ABG Intellectual Property Law, S.L.

(57) **Abstract**

The invention is comprised among apparatus and/or devices for manipulating and handling fluids. The invention relates to the integration of these apparatus and/or devices in a system for manipulating and/or handling any type of fluids. Specifically, the invention relates to a disposable cartridge and a platform cooperating with one another in a flexible system for manipulating fluids, as well as a flexible system for manipulating fluids. More particularly, the present invention is intended for culture cells.

## Description

### Object of the Invention

The invention is comprised among apparatus and/or devices for manipulating and handling fluids. The invention relates to the integration of these apparatus and/or devices in a system for manipulating and/or handling any type of fluids. Specifically, the invention relates to a disposable cartridge and a platform cooperating with one another in a flexible system for manipulating fluids, as well as a flexible system for manipulating fluids. More particularly, the present invention is intended for culturing cells.

### Background of the Invention

A specific system or equipment is designed today for each fluid handling and/or manipulating process to enable carrying out said process. More particularly, there are different bioreactors or systems today that are intended for processes of culturing or growing cells, each of said bioreactors or systems, as a whole, being specific for the culturing process and the type of cell to be cultured. In other words, there is no system that is flexible enough so as to allow culturing different types of cells, and the same occurs for other processes for handling and/or manipulating fluids. Processes for handling and/or manipulating fluids can be understood as cell culturing processes, chemical engineering processes, pharmaceutical production, etc.

To date, each culture system or bioreactor has been designed to solve a problem relating to culturing a specific type of cell, given that each cell type is cultured in a different way and under different physical and/or chemical conditions. Nevertheless, this practice has the drawback that for each process to be performed, the equipment or system must be designed entirely from scratch.

In each cell culture system or bioreactor, a large number of valves are normally used throughout the system, these valves being connected to a plurality of channels or tubes comprised in said system. These bioreactors have the drawback that in order to be able to operate the entire equipment or system, each tube or channel must be placed in its corresponding location and between each valve, in addition to the need to connect each of these valves with the system so that they are controlled by a computer. Known culturing systems therefore additionally have the drawback of being too manual and complex and being able lead to errors when making the numerous connections between components integrated in the cell culture system. In turn, these same drawbacks can be extrapolated to other processes for handling and/or manipulating fluids.

A flexible system which allows handling and/or manipulating any fluid has been developed to solve these technical problems. In other words, a platform cooperating with a disposable cartridge (which acts as a disposable cartridge) has been developed such that only a suitable expendable element is required for each process for handling and/or manipulating fluid, and a single platform would be used at all times for each process. Therefore, a flexible system which allows carrying out any process, particularly the culturing of any type of cell under any physical and/or chemical condition, is provided.

### Description of the Invention

The present invention proposes a solution to the preceding problems by means of a disposable cartridge for cooperating with a platform according to claim 1, a platform for cooperating with the disposable cartridge according to claim 11 and a flexible system for manipulating fluids according to claim 14. The dependent claims define the preferred embodiments of the invention.

A first inventive aspect provides a disposable cartridge for cooperating with a platform in a flexible system for handling and/or manipulating fluids, preferably a cell culture, characterized in that the disposable cartridge comprises:
at least one chamber suitable for storing and/or handling fluids therein,
a duct network configured for circulating fluids therein and for being in fluid communication with the interior of the at least one chamber, and
flow control means for controlling the flow of fluid circulating through the duct network, the flow control means for controlling the flow of fluid being configured for connecting with actuation means of the platform through a first multiconnector.

Throughout this document, "disposable cartridge" will be understood as a fixed element or device formed by a series of components including, among others, one or more chambers, ducts for fluid circulation, and flow control means for controlling the flow of fluid circulating through said ducts. Furthermore, this disposable cartridge is understood as a disposable element, i.e., an element that can be disposed of and replaced with another one once it is used and/or the fluid handling process ends.

The disposable cartridge that can be replaced with a new one which will cooperate with the same platform advantageously provides flexibility to the system for handling and/or manipulating fluids. In other words, it would not be necessary to design and manufacture an entire system for a particular fluid handling process, but rather different expendable elements cooperating with a platform for different processes for manipulating and handling fluids would be used.

The disposable cartridge comprises a chamber which is understood as a receptacle or reservoir in which fluids can be stored therein for handling and/or manipulating them by means of different processes for handling and/or manipulating fluids. More particularly, the chamber can be understood as a bioreaction chamber, or a chamber in which a chemical reaction is carried out, or in which a biological process is carried out.

The disposable cartridge further comprises a duct network which is formed by a plurality of ducts and/or tubes that can be in fluid communication with one another and/or with the interior of the chamber.

The flow control means for controlling the flow of fluids are configured for being operated by actuation means comprised in the platform. When the disposable cartridge cooperates with the platform, the fluid flow control means connect with the actuation means through a first multiconnector comprised in said disposable cartridge. "Connecting" will be understood herein as the action whereby two or more components come into communication. In other words, the fluid flow control means are configured for coming into communication with the actuation means of the platform.

The disposable cartridge is configured for cooperating with the platform in a flexible system for manipulating fluids. In other words, when the disposable cartridge cooperates with the platform, different components of the disposable cartridge connect or come into communication with the platform or components thereof. Furthermore, for this disposable cartridge to start cooperating with the platform or vice versa, the disposable cartridge must connect or come into communication with the platform, such as through the connection and/or coupling between a first multiconnector of the expendable element (disposable cartridge) and a second multiconnector of the platform, for example, for connecting the flow control means of the expendable element with the actuation means of the platform.

In a particular embodiment, the disposable cartridge further comprises fixing means configured for fixing the disposable cartridge to the platform. Advantageously, it can be fixed and/or coupled to and/or connected with the platform for a system for manipulating and/or handling fluids. In other words, as a result of the fixing means the disposable cartridge can be fixed to the platform.

In a particular embodiment, the flow control means for controlling the flow of fluid are configured for connecting, in fluid communication, with the actuation means through the first multiconnector. In other words, when the disposable cartridge cooperates with the platform, the flow control means for controlling the flow of fluid are operated by the actuation means comprised in the platform by means of a fluid connection through the first multiconnector. In other words, a fluid connection exists between the flow control means for controlling the flow of fluid and the platform, particularly, the actuation means, which means that they are in fluid communication with one another.

Advantageously, the presence of the first multiconnector in the disposable cartridge and of the second multiconnector in the platform, and of the coupling and/or connection existing between both, facilitates fluid connection of the tubes or connectors through which fluids (compressed air or nitrogen) of the disposable cartridge circulate with the actuation means of the platform providing compressed air or nitrogen, respectively.

In a more particular embodiment, the fluid communication between the flow control means for controlling the flow of fluid and the actuation means of the platform are compressed air or nitrogen. In other words, the actuation means operate the fluid flow control means of the disposable cartridge by means of compressed air or nitrogen.

In another particular embodiment, the flow control means for controlling the flow of fluid are configured for connecting, in electrical communication, with the actuation means through the first multiconnector. In other words, when the disposable cartridge cooperates with the platform, the flow control means are operated by the actuation means comprised in the platform by means of an electrical connection through the first multiconnector. In other words, an electrical connection exists between the flow control means for controlling the flow of fluid and the platform, particularly, the actuation means, which means that they are in electrical communication with one another.

The presence of the first multiconnector in the disposable cartridge, and of the second multiconnector in the platform, and of the coupling and/or connection existing between both, advantageously facilitates the electrical connection of the tubes or electrical connectors of the disposable cartridge with the actuation means of the platform providing said electrical communication.

In a particular embodiment, the disposable cartridge is a disposable cartridge for a system for culturing cells, wherein the at least one chamber is a bioreaction chamber, and wherein the fluids are culture media, or a liquid medium, or cells, or any combination thereof. In other words, in this particular embodiment, the process for handling and/or manipulating fluids is a cell culture process.

In a more particular example, the culture medium and the initial cells are initially housed in the interior of the at least one chamber to obtain a desired cell culture.

In a particular embodiment, the disposable cartridge comprises a plurality of receptacles connected to the duct network suitable for storing fluids therein and configured for being in fluid communication with one another and/or with the interior of the at least one chamber.

Each of the receptacles of said plurality comprised in the disposable cartridge are understood as storage reservoirs or chambers, each having a different use depending on its application, i.e., depending on the fluid handling process involved. Advantageously, the present disposable cartridge gives the user total freedom to design a fluid handling process and to carry it out with a single piece of equipment, i.e., with a single system for manipulating and/or handling fluids.

In a more particular embodiment, the disposable cartridge comprises a residue receptacle connected to the duct network configured for housing therein fluid residues coming from the at least one chamber. In another particular embodiment, the disposable cartridge comprises a reservoir configured for storing a fluid or an end product, said reservoir being in fluid communication with the interior of the at least one chamber through the duct network.

The residue receptacle advantageously allows storing therein fluids circulating through the duct network, as well as fluids coming from the interior of the chamber, or even other fluids coming from other receptacles comprised in the interior of the disposable cartridge, such as a cleaning liquid coming from a cleaning receptacle, for example. The residue receptacle is in fluid communication with the interior of the at least one chamber through the duct network. Furthermore, this residue receptacle is suitable for storing therein leftover fluids or fluids that are negligible in the process for handling or manipulating fluids, or fluids that are not of interest for the user.

In a particular example of cell culture, the leftover culture medium or culture medium residues coming from the chamber are delivered to said residue receptacle.

The reservoir is understood as a final receptacle in which a fluid or an end product is stored, i.e., a resulting fluid, an end product, or part thereof which is of interest after handling and/or manipulating the initial fluid or fluids that were initially in the interior of said chamber, or introduced into this chamber for the process for handling and/or manipulating at least one fluid. Advantageously, a fluid or a combination of several fluids can be obtained directly from the reservoir as an end product without having to perform an additional step for obtaining the desired end product. In a particular example for cell culture, the desired end product or fluid will be understood as the cultured cells, or the final cells, and/or a culture medium, and it will be housed in a reservoir.

In a particular embodiment, the at least one chamber is configured for receiving at least a first initial product coming from a first prechamber through the duct network. For a particular example of cell culture, a first initial product can be understood as the initial cells or initial cells and a medium.

In a more particular embodiment, the at least one chamber is furthermore configured for receiving a second initial product coming from a second prechamber by means of the duct network, preferably through the first prechamber. In other words, the second prechamber can be in direct fluid connection with the interior of the at least one chamber. However, the second prechamber would preferably be in fluid communication with the first prechamber, i.e., the second initial product is initially introduced into the interior of the first prechamber to then be conveyed together with the first initial product into the interior of the at least one chamber through the duct network. For a particular example of cell culture, a second initial product can be understood as a culture medium, i.e., a medium suitable for culture cells.

In a particular embodiment, the disposable cartridge comprises the at least a first prechamber suitable for storing at least a first initial product, wherein this first prechamber comprises a first inlet for introducing therein the first initial product.

At least a first fluid or initial product is initially housed in this first prechamber, i.e., before the process for handling and/or manipulating fluids, to then be circulated or conveyed through the duct network to the interior of the chamber for subsequent handling or manipulation.

In this particular embodiment, the first prechamber is located in the disposable cartridge. However, in another particular embodiment, the first prechamber is not part of the disposable cartridge, and it can be arranged in the platform or outside the system for handling and/or manipulating fluids.

In another more particular embodiment, the first prechamber initially contains at least a first initial product, and the at least one chamber contains a second initial product. In other words, when the first initial product coming from the first prechamber is introduced into the interior of the chamber, it reacts with a second initial product, giving rise to the handling or manipulation of fluids (products). Nevertheless, the presence of a first prechamber advantageously allows the at least one initial product to be housed initially in a receptacle other than the at least one chamber in which fluids are to be handled and/or manipulated, and this thereby prevents the handling of fluids from starting before it is desired or in a sudden manner.

In a more particular embodiment, the first prechamber comprises a second inlet for introducing a second initial product coming from a second prechamber. In a more particular embodiment, the second prechamber is located in the disposable cartridge. In another particular embodiment, the second prechamber is located in the platform. In a particular example of cell culture, it is understood that the culture medium contained in a second prechamber can be introduced into the first prechamber together with the initial cells or initial cells with a medium, before the cell culture process is started, i.e., before both initial products are introduced into the interior of the chamber and the handling and/or manipulation of fluids starts.

In another particular embodiment, the disposable cartridge comprises at least one sensor connected to the duct network, the at least one sensor being configured for measuring at least one fluid parameter.

The at least one sensor comprised in the disposable cartridge is configured for connecting with the processing means of the control unit of the platform. Advantageously, the presence of one or more sensors allows controlling the handling of at least one fluid contained in the chamber and/or circulating through the duct network. In the particular example of cell culture, these sensors allow controlling the state of the cells and the culture medium during the cell culture process carried out in the interior of the bioreaction chamber.

In a particular embodiment, the disposable cartridge comprises a cleaning receptacle connected to the duct network, the cleaning receptacle being configured for releasing a cleaning liquid from its interior along the duct network.

In a particular embodiment, the disposable cartridge comprises conditioning means configured for conditioning at least one of the initial products before being introduced into the interior of the at least one chamber. These conditioning means can condition the pH of the fluid, or the concentration of O₂ dissolved in the fluid, or the ionic force of this fluid, among others. Advantageously, the initial fluid can be conditioned through these conditioning means before entering the interior of the at least one chamber.

In a more particular embodiment, the conditioning means are heating means configured for heating at least one of the initial products before being introduced into the interior of the at least one chamber. In other words, if one of the initial products is contained in a prechamber at a specific temperature or these same initial products are themselves at a specific temperature, preferably less than the initial desired temperature, the temperature of said initial product can advantageously be modified through these heating means before being introduced into the interior of the at least one chamber. In a more particular embodiment, the heating means are a heater. For a particular example of cell culture, if the culture medium is subjected to a temperature lower than the desired temperature, this culture medium can be heated through these heating means. Nevertheless, since the culture medium is introduced into the first prechamber or directly into the interior of the bioreaction chamber, this culture medium of interest for culture cells must be heated before it comes into contact with the initial cells.

In a particular embodiment, the flow control means for controlling the flow of fluid are:
valves, or
pumps, or
a combination of the above.

In a more particular embodiment, the valves are tube pinch valves and/or solenoid valves. Advantageously, the use of valves of this type does not interfere with the tube or duct.

In another more particular embodiment, the pumps are peristaltic micropumps. The peristaltic micropumps are configured for driving or circulating fluids through the ducts and they can furthermore act as conventional pumps or valves. Furthermore, micropumps of this type may be disposable.

In a particular embodiment, the system comprises means for driving the circulation of the fluid contained in the at least one chamber through the duct network. In a more particular embodiment, the means for driving the circulation of fluid are a peristaltic pump, or compressed air injection, or communicating vessels, or a combination thereof. This peristaltic pump for driving fluid contained in the chamber is mainly formed by a motor, which is understood as the actuator, and a head with an inlet and an outlet connecting with the ducts of the actual duct network of the disposable cartridge. In particular, the pump motor is located in the platform and the pump heads the inlet and outlet of which are connected with the duct network are part of the disposable cartridge itself. In a particular embodiment in which the disposable cartridge is coupled to the platform, said head of the disposable cartridge is connected and/or coupled to the motor arranged in the platform. In a more particular embodiment, the at least one peristaltic pump, particularly the pump motor, is configured for being fixed to a fixing structure of the platform through fixing means.

In a particular embodiment, the disposable cartridge comprises a housing structure in which the at least one chamber, part of the duct network and the flow control means for controlling the flow of fluid are housed, wherein said housing structure is configured for being fixed to a first structure, which can be understood as a male structure, of the platform through the fixing means. In a more particular embodiment, these fixing means are a second structure, which can be understood as a female structure, configured for being fixed and/or coupled to the first structure, i.e., the male structure of the platform.

Advantageously, the housing structure allows being easily integrated in the platform, so the connection and/or integration of the disposable cartridge with the platform is simplified.

In another particular embodiment, the residue receptacle and the reservoir are located outside the housing structure and are configured for being housed in a second compartment of the platform, wherein the residue receptacle and the reservoir are both in fluid connection with the interior of the housing structure through external ducts connected to the duct network or the interior of the chamber and adapted for being connected by means of connectors. In a particular case of cell culture, said connectors are sterile.

The fact that both the reservoir and the residue receptacle is located outside the housing structure advantageously allows, in processes in which more than one final fluid or excess fluid residues may be obtained, being able to replace both the receptacles, i.e., the reservoir and the residue receptacle, with new ones. Furthermore, in the particular embodiment of cell culture or in other processes in which it is very important not to contaminate the fluids circulating through the system from the outside, the fact that both the reservoir and the residue receptacle may be replaced with a new one as a result of the sterile connectors results in this replacement or substitution of receptacles not interfering with the disposable cartridge.

In another particular embodiment, the heating means are configured for being housed in the second compartment of the platform.

In another particular embodiment, the housing structure is configured for comprising therein a temperature higher than the temperature of the storage module of the fixed part.

In a second inventive aspect, the invention provides a platform for cooperating with the disposable cartridge of any of the embodiments of the first inventive aspect, in a flexible system for handling and/or manipulating fluids, preferably a cell culture, characterized in that it comprises:
actuation means adapted for acting on the flow control means for controlling the flow of fluid of the disposable cartridge,
a second multiconnector configured for connecting a first multiconnector of the disposable cartridge and the actuation means, and
a control unit configured for controlling the operation of the actuation means and the disposable cartridge.

The second multiconnector is coupled to or connects with the first multiconnector of the disposable cartridge when it cooperates with the platform. Advantageously, these multiconnectors allow the connections of each of the flow control means for controlling the flow of fluid with the actuation means to be centralized through said multiconnectors, which in turn allows a simple and quick connection between the disposable cartridge and the platform as a result of the coupling of these multiconnectors.

The control unit is in charge of controlling both the actuation means and the flow control means for controlling the flow of fluid located in the disposable cartridge and operated by the actuation means.

In a particular embodiment, the platform comprises a first structure, which can be understood as a male structure configured for fixing thereon the disposable cartridge, preferably the housing structure thereof.

In a particular embodiment, the platform is a platform for a system for culture cells. In other words, in this particular embodiment, the process for handling and/or manipulating fluids is a cell culture process.

In a particular embodiment, the actuation means are gas distribution means and they are configured for being in fluid communication with the disposable cartridge through the first and second multiconnectors when the flow control means for controlling the flow of fluid of the disposable cartridge are configured for connecting, in fluid communication, with the actuation means through the first multiconnector. More particularly, the gas distribution means are means for distributing compressed air or nitrogen or air enriched with CO₂.

In a particular embodiment, the actuation means are electric current distribution means and they are configured for being in electrical communication with the disposable cartridge through the first and second multiconnectors when the flow control means for controlling the flow of fluid of the disposable cartridge are configured for connecting, in electrical communication, with the actuation means through the first multiconnector.

In a particular embodiment, the platform comprises a storage module configured for being in fluid communication with the disposable cartridge and for maintaining a pre-established temperature therein. In a more particular embodiment, the storage module comprises a second prechamber suitable for storing a second initial product, this second prechamber being configured for being in fluid communication with the disposable cartridge. In the particular example of cell culture, the culture medium can be stored in the second prechamber which is located in the storage module at a temperature lower than the temperature of the interior of the disposable cartridge.

In a particular embodiment, when the disposable cartridge comprises a pump for driving an inflow fluid, the platform further comprises a pump motor configured for being connected or coupled to a pump head arranged in the disposable cartridge. In particular, the pump motor is operated by the control unit of the platform.

In a third inventive aspect, the invention provides a flexible system for handling and/or manipulating fluids, preferably a cell culture, comprising:
a disposable cartridge according to any of the embodiments of the first inventive aspect, and
a platform according to any of the embodiments of the second inventive aspect.

This system is advantageously interesting from the flexibility viewpoint as it allows performing different processes for handling and/or manipulating fluids by integrating a disposable cartridge with a platform. In other words, depending on the process to be performed, the disposable cartridge required according to the desired process only has to be integrated in or connected to the platform. Furthermore, in some fluid handling processes, the process itself changes, i.e., it has steps which, in order to be carried out, require the system to have special components that were already initially included in the expendable element and must be replaced, or they require incorporating new components to said expendable element, or even to the platform. In this manner, changing the expendable element and redesigning its components or platform components would be enough in order to continue working in a specific process.

The system is easy to install and use, which advantageously reduces connection failure between the components, where it is only necessary to couple the disposable cartridge to the platform and start the process for handling and/or manipulating fluids.

In a particular embodiment, the flexible system for handling and/or manipulating fluids is a flexible system for a cell culture.

In other words, any fluid process required can be controlled and performed with one and the same platform. For example, different types of cells can be cultured without having to design an entire cell culture system or bioreactor for a specific cell culture process. Likewise, by only changing the disposable cartridge which is coupled to the platform for cooperating therewith, any type of cell can be cultured in any fluid dynamic configuration that is desired.

All the features and/or steps of methods described in this specification (including the claims, description and drawings) can be combined in any combination, with the exception of combinations of such mutually exclusive features.

### Description of the Drawings

These and other features and advantages of the invention will be more clearly understood based on the following detailed description of a preferred embodiment given only by way of illustrative and non-limiting example in reference to the attached drawings.
Figure 1A schematically shows a particular example of a flexible system according to the present invention.
Figure 1B schematically shows a particular example of a flexible system according to the present invention.
Figure 2 shows a perspective view of the platform according to a particular example of the present invention.
Figure 3A shows a perspective view of the housing structure of the disposable cartridge according to a particular example of the present invention.
Figure 3B shows a rear view of the housing structure of the disposable cartridge according to a particular example of the present invention.
Figure 4 shows a perspective view of a detail of the flexible system according to a particular example of the present invention.
Figure 5 shows a perspective view of a detail of the flexible system according to another particular example of the present invention.
Figure 6 schematically shows a particular example of a section of a flexible system according to the present invention.
Figure 7 schematically shows a particular example of a section of a flexible system according to the present invention.

### Detailed Description of the Invention

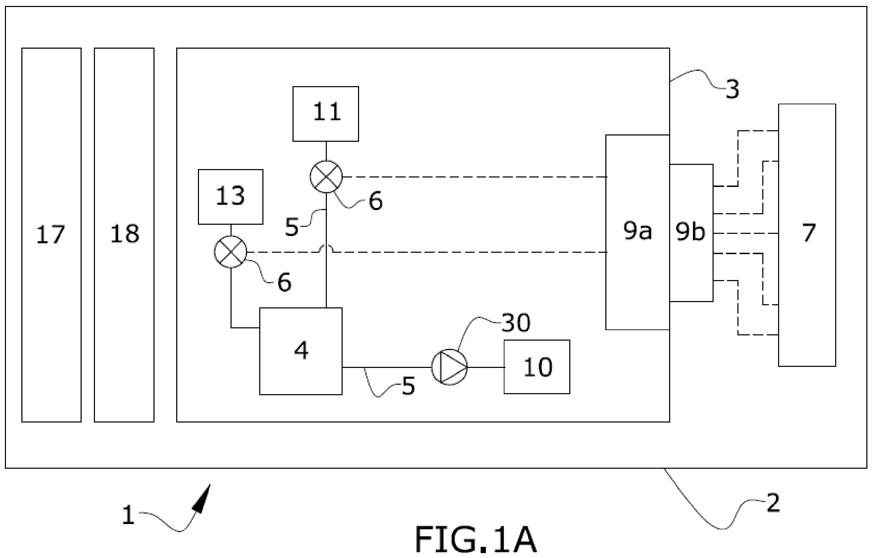

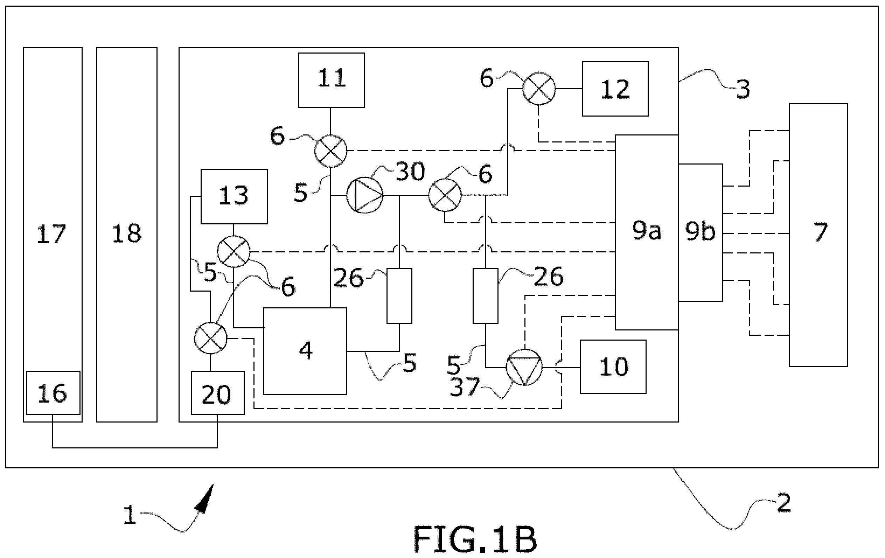

Figures 1 to 5 show a particular example of the present invention in which the flexible system (1) is a system for culturing cells. In this particular example, the chamber (4) is a bioreaction chamber and the fluids are culture media, culture media with cells and/or cleaning liquid.

Figures 1A to 1B schematically show a system (1) for culturing cells, the system (1) comprising a platform (2) and a disposable cartridge (3) cooperating with one another.

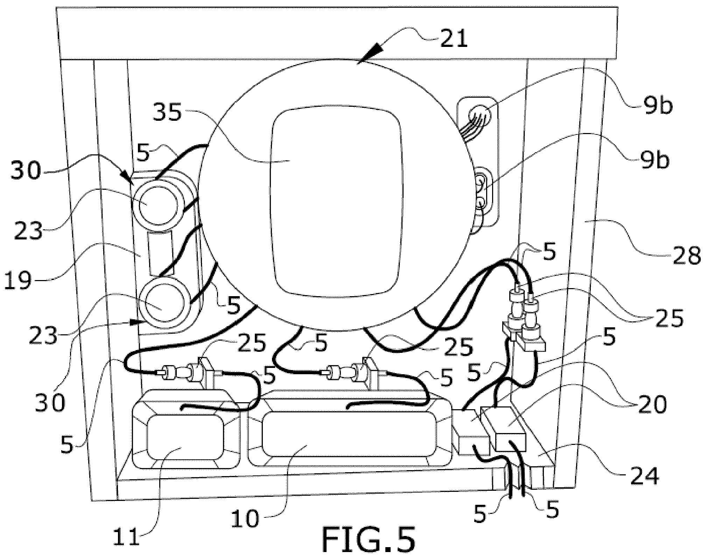

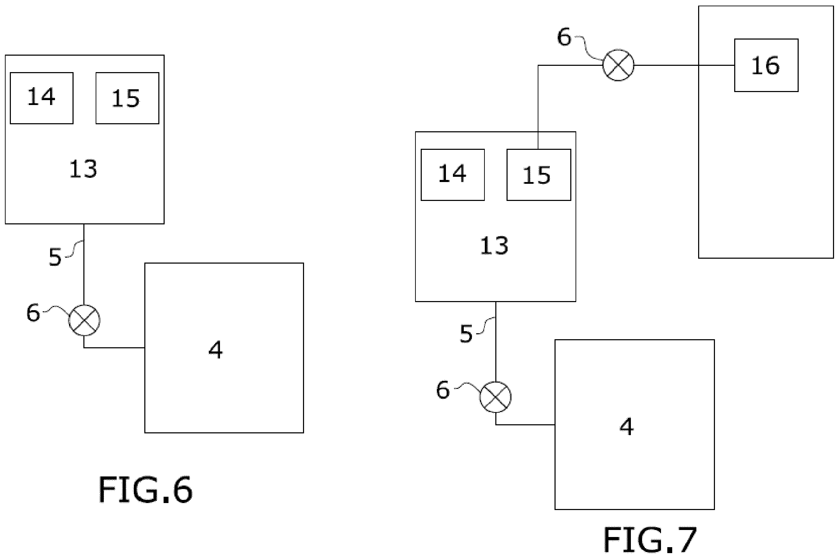

In particular, the disposable cartridge (3) shown in Figures 1A to 1B has a bioreaction chamber (4) for culturing cells which is in fluid communication with a first prechamber (13) in which an initial product, such as a culture medium and the cells suspended therein, for example, is stored. Furthermore, the chamber (4) is in fluid communication with a first reservoir (11) in which the desired end product, such as the cells cultured in a culture medium, for example, is stored. In turn, the bioreaction chamber (4) is in fluid communication with a residue receptacle (10) in which leftover residual fluids coming from the bioreaction chamber (4), such as leftover culture medium, for example, are stored. In particular, Figure 1B furthermore shows a cleaning receptacle (12) in which the cleaning liquid or PBS is stored. In this particular example, the prechamber (13) has a first inlet (14) through which the cells are introduced, and a second inlet (15) through which the culture medium is introduced (as shown in Figure 7). The second inlet (15) is in fluid communication with a second prechamber (16) arranged in the storage module (17) of the platform (2) (as shown in Figure 1B). Before the culture medium coming from the second prechamber (16) is introduced into the first prechamber (13), it will pass through heating means (20) which heat this culture medium, i.e., they change its temperature for introducing it into said first prechamber (13) together with the initial cells. In Figures 1A to 1B, the chamber (4) and the different receptacles or devices arranged in the shown systems are in fluid communication with one another through a duct network (5) formed by a plurality of tubes or ducts. Each of the flow control means for controlling the flow of fluid is connected with a first multiconnector (9a) through tubes or ducts through which compressed air coming from actuation means (7) located in the platform (2) circulates. These flow control means for controlling the flow of fluid are a combination of valves (6) and pumps (30, 37). In these examples in particular, the valves (6) are pinch valves and the pumps (30, 37) are peristaltic pumps for driving the fluid (30) and/or micropumps (6). In particular, the micropumps (37) are part of the disposable cartridge and are in fluid communication with the first multiconnector (9a), whereas the peristaltic pumps (30) for driving the fluid are formed by pump motors (36) that are located in the platform (2) (not shown in the drawing) and by pump heads (23) that are located in the disposable cartridge (3) (as shown in detail in Figure 5) and coupled to said pump motors (36) .

The first multiconnector (9a) arranged in the disposable cartridge (3) is coupled to the second multiconnector (9b) arranged in the platform (2). Furthermore, the second multiconnector (9b) is connected to the actuation means (7) by means of tubes or ducts through which compressed air that will be conveyed to the flow control means of the disposable cartridge (3) for operating same circulates.

The platform (2) shown in both Figures 1A to 1B furthermore has a control unit (18) controlling the disposable cartridge (3), the actuation means (7), and the storage module (17).

In a particular example of cell culture, the storage module (17) has therein a temperature of 4°C, whereas the housing structure (21) in which the disposable cartridge (3) is housed has a temperature of 37°C.

In particular, the flexible system (1) shown in Figure 1B furthermore has two sensors (26) located in the duct network (5). These sensors (26) are configured for detecting properties of interest of the fluid circulating through the duct network (5) .

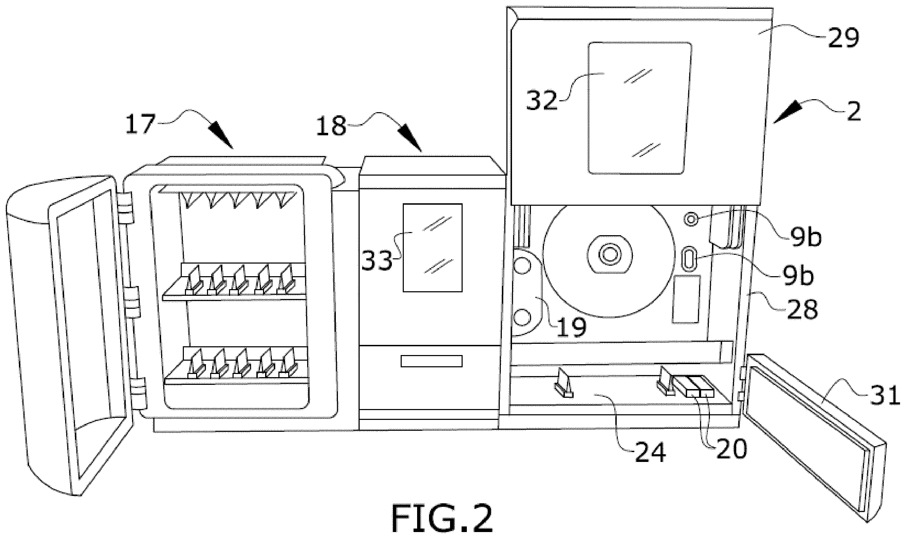

Figure 2 shows a perspective view of the platform (2) of a system (1) for the cell culture without any of the components of the disposable cartridge (3). As seen in said drawing, the platform (2) comprises a storage module (17) arranged at one end of the platform (2), this storage module (17) being understood as a refrigerator as it stores therein, for example culture medium, cleaning liquid or other fluids, at a specific temperature, which is preferably lower than the temperature of the disposable cartridge (3) or the structure or compartment in which the disposable cartridge (3) is fixed to the platform (2).

The platform (2) furthermore has at the other end thereof a first compartment (28) configured for housing the disposable cartridge (3) therein. In turn, the platform (2) comprises in a lower part with respect to the first compartment (28), a second compartment (24) suitable for accommodating the first reservoir (11), the residue receptacle (10) and the heating means (20). The first compartment (28) of the platform (2) comprises a male structure (22) on which the housing structure (21) of the disposable cartridge (3) will be fixed through its female structure (8) (fixing means). Furthermore, the first compartment (28) of the platform (2) comprises a fixing structure (19) in which there are located motors (36) (not shown) of the peristaltic pumps (30) on which the heads (23) of said peristaltic pumps (30) of the disposable cartridge (3) are coupled or fixed (as seen in detail in Figure 5). This first compartment (28) further comprises the actuation means (7) (not shown in the drawing) which are in fluid communication with the flow control means of the disposable cartridge (3) through the first multiconnector (9a) (not shown in the drawing) and the second multiconnector (9b). In this particular example, the platform (2) has two second multiconnectors (9b).

The first compartment (28) of the platform (2) has a first door (29) closing said first compartment (28). In turn, the second compartment (24) has a second door (31) closing this second compartment (24). So each compartment (24, 28) can be accessed independently of one another, such that it advantageously allows extracting, for example, the end product from the reservoir (11) or the residues stored in the residue receptacle (10) without having to interfere with the first compartment (28). In particular, the first door (29) of the first compartment (28) has a first window (32) which advantageously allows being able to see the interior of the compartment during the cell culture process.

Likewise, the platform (2) has a control unit (18) arranged between the storage module (17) and the first compartment (28). The control unit (18) comprises a display (33) on which information about the cell culture is shown, in addition to information relating to the storage module (17) and the first compartment (28).

In a particular example, the platform comprises the storage module (17), a control unit (18) and a plurality of first compartment (28) and second compartment (24). Different cell culture processes can therefore be carried out at the same time, all of them being controlled by the control unit (18).

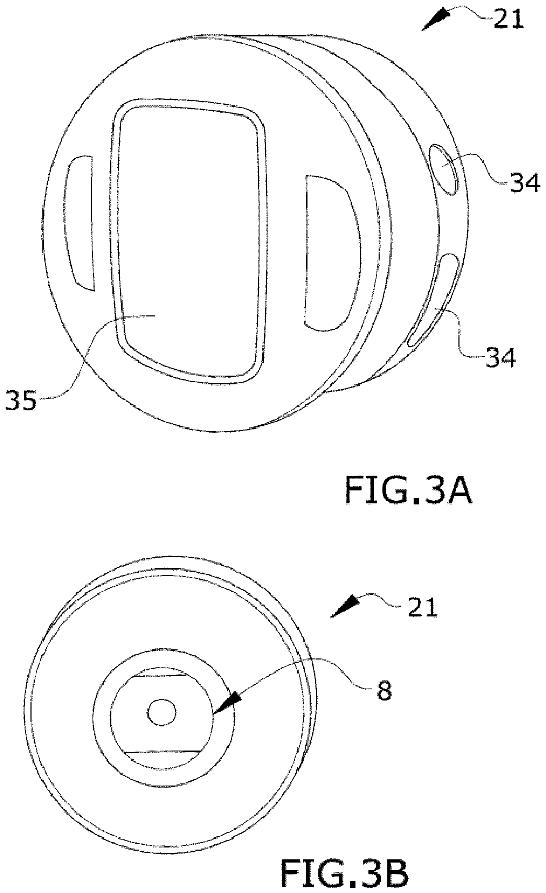

Figures 3A to 3B show a housing structure (21) of the disposable cartridge (3) in which the chamber (4), among other components (not shown in the drawings), is housed. Figure 3A shows a perspective view of said housing structure (21), where it can be observed in more detail that this structure comprises a series of holes (34) through which the different connections and/or communication of the disposable cartridge (3) with the platform (2) and with the actual components integrated in said disposable cartridge (3) are made. The housing structure (21) comprises a first part in which the holes (34) are arranged, and a second part which is understood as a cover of the housing structure (21). This cover or second part of the housing structure has a second window (35) through which the interior of said housing structure (21), and accordingly the elements integrated therein, can be seen. Furthermore, this second part or cover of the housing structure (21) has closure means through which the second parte closes and/or fits together with the first part such that the housing structure (21) appears as a closed structure or compartment. Figure 3B shows a rear view of the housing structure (21), particularly the first part of this structure, in which the female structure (8) (fixing means) which is configured for being coupled and/or fixed to the male structure (22) of the platform (2), can be seen.

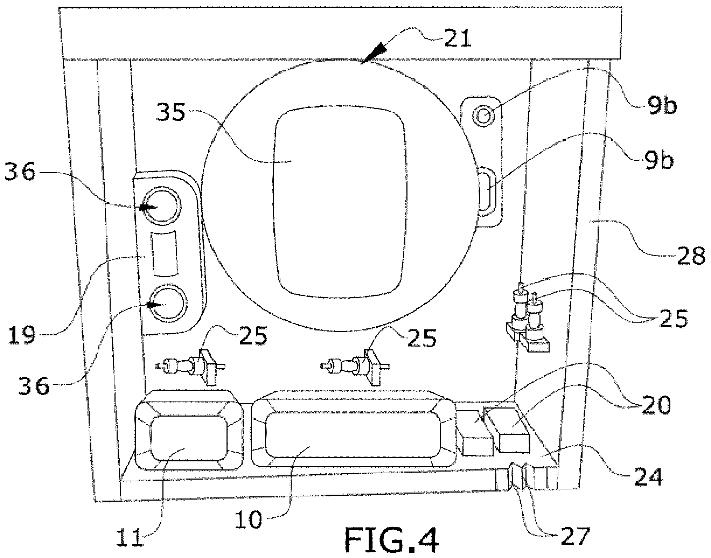

Figures 4 to 5 show perspective views of the compartments (24, 28) of the platform (2) and the disposable cartridge (3) fixed to said platform (2). The manner in which the housing structure (21) is coupled to the platform (2) in the first compartment (28) thereof can be seen in both drawings. There can be seen in this first compartment (28) two second multiconnectors (9b) arranged one after another, in addition to two heads (23) of peristaltic pumps (30) coupled on the pump motors (36) located in the fixing structure (19) of the platform (2). These drawings furthermore show a second compartment (24) in which the first reservoir (11), the residue receptacle (10), and the heating means (20) are located. In turn, the second compartment (24) has through grooves (27) through which the tubes and/or ducts that are part of the duct network (5) pass, said tubes and/or ducts putting the second prechamber (16), arranged in the storage module (17) (not shown), in fluid communication with the disposable cartridge (3) through the heating means (20). Furthermore, as shown in both Figures 4 and 5, two sterile connectors (25) that are part of the disposable cartridge (3) and are suitable for keeping the fluid communication along the duct network (5) in the system (1) sterile are coupled to the first compartment (28).

Figure 5 shows the same perspective as Figure 4 and the different connections and/or communications between the components integrated in the compartments (24, 28). Two heads (23) of peristaltic pumps (30) of the disposable cartridge (3) which are coupled to a fixing structure (19) of the platform (2) in which the pump motors are located can be seen in this Figure 5. These pump heads (23) are in fluid communication with the interior of the housing structure (21) by means of the duct network (5) through the holes (34). It can be seen in particular how the each pump head (23) has a duct inlet and a duct outlet of the duct network (5) of the disposable cartridge (3). Figure 5 furthermore shows two first multiconnectors (9a) each coupled to a second multiconnector (9b), and how these multiconnectors are in fluid communication with the interior of the housing structure (21) through a series of tubes and/or ducts through which compressed air coming from the actuation means (7) circulates. In this particular example, the actuation means (7) are compressed air distribution means, such as a compressed air distribution rack, for example.

Figure 5 also shows how both the residue receptacle (10) and the reservoir (11) are in fluid communication, independently of one another, with the interior of the housing structure (21) by means of the duct network (5) through the holes (34). These ducts (5) communicating said receptacles (10, 11) with the interior of the housing structure must be sterile, therefore there are sterile connectors (25) connected to each duct (5) to thereby keep said communication sterile.

Likewise, Figure 5 shows how the heating means (20) are in fluid communication with the interior of the housing structure (21) by means of the duct network (5) through the holes (34) (shown in Figure 3A).

Figures 6 and 7 schematically show the first prechamber (13) having a first inlet (14) through which the initial cells are introduced into the interior of the first prechamber (13), and a second inlet (15) through which the culture medium is introduced into the interior of the first prechamber (13). Furthermore, it shows how the first prechamber (13) is connected to the chamber (4) through the duct network (5), particularly through a tube/duct on which a pinch valve (6) is located for regulating the passage of cells suspended in the culture medium to the interior of the chamber (4).

Figure 7 furthermore shows how the second inlet (15) is connected with a second prechamber (16) which is located in a storage module (17) arranged in the platform (2), this second prechamber (16) storing therein culture medium under specific temperature conditions. The connection between the second inlet (15) and the second prechamber (16) is carried out through the duct network (5), particularly through a tube/duct on which a pinch valve (6) is located for regulating the passage of the culture medium to the second inlet (15).

## Claims

1. A disposable cartridge (3) for cooperating with a platform (2) in a flexible system (1) for handling and/or manipulating fluids, preferably a cell culture, **characterized in that** the disposable cartridge (3) comprises:
at least one chamber (4) suitable for storing and/or handling fluids therein,
a duct network (5) configured for circulating fluids therein and for being in fluid communication with the interior of the at least one chamber (4), and
flow control means for controlling the flow of fluid circulating through the duct network (5), the flow control means for controlling the flow of fluid being configured for connecting with actuation means (7) of the platform (2) through a first multiconnector (9a).

2. The disposable cartridge (3) according to claim 1, **characterized in that** the flow control means for controlling the flow of fluid are configured for connecting, in fluid communication, with the actuation means (7) of the platform (2) through the first multiconnector (9a).

3. The disposable cartridge (3) according to claim 1, **characterized in that** the flow control means for controlling the flow of fluid are configured for connecting, in electrical communication, with the actuation means (7) of the platform (2) through the first multiconnector (9a).

4. The disposable cartridge (3) according to any of the preceding claims, **characterized in that** it comprises a plurality of receptacles connected to the duct network (5) suitable for storing fluids therein and configured for being in fluid communication with one another and/or with the interior of the at least one chamber (4).

5. The disposable cartridge (3) according to any of the preceding claims, **characterized in that** the at least one chamber (4) is configured for receiving at least a first initial product coming from a first prechamber (13) through the duct network (5) .

6. The disposable cartridge (3) according to claim 5, **characterized in that** the at least one chamber (4) is configured for receiving a second initial product coming from a second prechamber (16) by means of the duct network (5), preferably through the first prechamber (13).

7. The disposable cartridge (3) according to any of claims 5 or 6, **characterized in that** it comprises the at least a first prechamber (13) suitable for storing at least a first initial product, wherein this first prechamber (13) comprises a first inlet (14) for introducing therein the first initial product.

8. The disposable cartridge (3) according to claims 6 and 7, **characterized in that** the first prechamber (13) comprises a second inlet (15) for introducing a second initial product coming from the second prechamber (16).

9. The disposable cartridge (3) according to any of claims 7 to 8, **characterized in that** it comprises conditioning means (20) configured for conditioning at least one of the initial products before being introduced into the interior of the at least one chamber (4).

10. The disposable cartridge (3) according to any of the preceding claims, **characterized in that** it comprises means for driving the circulation of the fluid contained in the at least one chamber (4) through the duct network (5).

11. A platform (2) for cooperating with the disposable cartridge (3) according to any of claims 1 to 10, in a flexible system (1) for handling and/or manipulating fluids, preferably a cell culture, **characterized in that** it comprises:
actuation means (7) adapted for acting on the flow control means for controlling the flow of fluid of the disposable cartridge (3),
a second multiconnector (9b) configured for connecting the first multiconnector (9a) of the disposable cartridge (3) and the actuation means (7), and
a control unit (18) configured for controlling the operation of the actuation means (7) and the disposable cartridge (3).

12. The platform (2) according to claim 11, **characterized in that** the actuation means (7) are gas distribution means and they are configured for being in fluid communication with the disposable cartridge (3) through the first multiconnector (9a) and second multiconnector (9b) when the disposable cartridge (3) is according to claim 2.

13. The platform (2) according to claim 11, **characterized in that** the actuation means (7) are electric current distribution means and they are configured for being in electrical communication with the disposable cartridge (3) through the first multiconnector (9a) and second multiconnector (9b) when the disposable cartridge (3) is according to claim 3.

14. The platform (2) according to any of claims 11 to 13, **characterized in that** it comprises a storage module (17) configured for being in fluid communication with the disposable cartridge (3) and for maintaining a pre-established temperature therein.

15. A flexible system (1) for handling and/or manipulating fluids, preferably a cell culture, comprising:
a disposable cartridge (3) according to any of claims 1 to 10, and
a platform (2) according to any of claims 11 to 14.
